# EUROPÄISCHE PATENTSCHRIFT

(11) **EP 2 515 852 B1**
(45) Veröffentlichungstag und Bekanntmachung des Hinweises auf die Patenterteilung: **02.03.2016**
(21) Anmeldenummer: 10798015.3
(22) Anmeldetag: 22.12.2010
(51) Int. Cl.: A61K 9/00, A61K 9/16, A61K 9/20, A61K 31/519

(54) **ORAL DISPERSIBLE TABLETTE ENTHALTEND KOMPAKTIERTE SILDENAFIL-BASE**
FUSION TABLET CONTAINING COMPACTED SILDENAFIL BASE
COMPRIMÉ ORODISPERSIBLE CONTENANT UNE BASE DE SILDENAFIL COMPACTÉE

(30) Priorität: 23.12.2009 EP 09015973
(43) Veröffentlichungstag der Anmeldung: 31.10.2012
(73) Patentinhaber: ratiopharm GmbH, 89079 Ulm (DE)
(72) Erfinder: WINTER, Sven, 59269 Neubeckum (DE); SCHEIWE, Max-Werner, 79689 Maulburg (DE); SWATSCHEK, Dieter, 89143 Blaubeuren (DE)
(74) Vertreter: Ter Meer Steinmeister & Partner
(86) Internationale Anmeldenummer: PCT/EP2010/007891
(87) Internationale Veröffentlichungsnummer: WO 2011/076411

(56) Entgegenhaltungen:
- EP-A1- 1 120 120
- EP-A2- 0 960 621
- WO-A1-2009/123626
- US-B1- 6 221 402

## Beschreibung

Die Erfindung betrifft ein Verfahren wie durch die Ansprüche definiert zur Herstellung eines pharmazeutischen Intermediats, umfassend die Schritte (i) Mischen von (a-i) Sildenafil-Base, (b-i) Dochtmittel, (c-i) Sprengmittel, (d-i) gegebenenfalls Fließmittel; (ii) Kompaktieren der Mischung; und (iii) Zerkleinern des Kompaktats, und ein durch dieses Verfahren erhältliches Intermediat. Ferner betrifft die Erfindung ein Verfahren zur Herstellung einer oral dispersiblen Tablette (nachfolgende auch als "Schmelztablette" bezeichnet), umfassend das erfindungsgemäße Intermediat sowie durch dieses Verfahren erhältliche Schmelztabletten.

Der IUPAC-Name von Sildenafil [INN] ist 1-{[3-(1-Methyl-7-oxo-3-propyl-6,7-dihydro-1H-pyrazolo-[4,3-d]pyrimidin-5-yl)-4-ethoxyphenyl]-sulfonyl}-4-methylpiperazin. Die chemische Struktur von Sildenafil wird in nachstehender Formel (1) dargestellt:

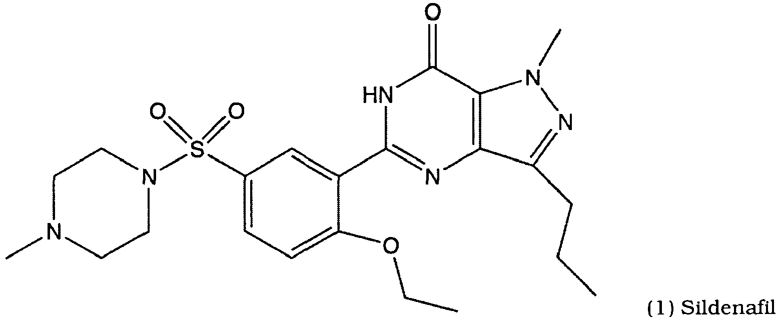

Sildenafil ist ein potenter selektiver Hemmer der cGMP-spezifischen Phosphodiesterase vom Typ 5 (PDE-5), die für die Herabsetzung von cGMP im Corpus cavernosum verantwortlich ist. Sildenafil in der Form seines Salzes Citrat wird unter dem Handelsnamen Viagra^{®} zur Behandlung der erektilen Dysfunktion vertrieben.

Im Stand der Technik werden verschiedene Verfahren zur Herstellung einer Schmelztablette enthaltend Sildenafil vorgeschlagen. EP 0 960 621 A2 beschreibt die Herstellung von porösen Schmelztabletten, enthaltend Sildenafil, wobei ein wasserlösliches, schmelzbares Bindemittel, mindestens ein Hilfsstoff und Sildenafil zunächst zu einer Tablette geformt werden und anschließend das Bindemittel geschmolzen und verfestigt wird.

EP 1 120 120 A1 beschreibt ein Verfahren zur Herstellung einer Schmelztablette, das die Vermischung eines cyclischen GMP Phosphodiesterase-Hemmer mit einem Saccharid, das Kneten dieser Mischung mit einem organischen Lösungsmittel, Wasser oder einem wässrigen organischen Lösungsmittel und dem anschließenden Formpressen in eine Schmelztablette umfasst.

WO 2009/123626 beschreibt oral dispersible Tabletten mit PDE-5 Inhibitoren, wobei zur Formulierung bis zu 50 Gew.-% an Kationenaustauscherharz erforderlich ist, was sich negativ auf das Mundgefühl auswirken kann. Zudem sind die beschriebenen Sildenafil Formulierungen aufwendig in der Herstellung, da eine zweifache Naßgranulation erforderlich ist (1. Naßgranulation: Sildenafil + Kationenaustauscherharz, 2. Naßgranulation: Hilfsstoffe).

Die im Stand der Technik beschriebenen Verfahren zur Herstellung von Schmelztabletten sind aufwendig und ermöglichen lediglich Schmelztabletten, die einen Wirkstoffgehalt von deutlich weniger als 50 Gew.-% aufweisen. Ein derart geringer Wirkstoffgehalt führt allerdings bei gleicher Dosierung zu einem höheren Tablettengewicht, was sich oftmals problematisch auf die Tabletteneigenschaften auswirken kann. Schmelztabletten mit einem zu hohen Tablettengewicht (z.B. größer 300 mg) werden von Patienten oftmals abgelehnt, da sich nach Zerfall der Schmelztablette eine große Menge an Tablettiermasse im Mundraum befindet, die als störend und unangenehm empfunden wird. Zerfallszeit und Mundgefühl der gemäß dem Stand der Technik erhältlichen Tabletten sind verbesserungsbedürftig. Ein Wirkstoffgehalt von mehr als 45 % oder sogar mehr als 50 % ist mit dem beschriebenen Verfahren nicht vorteilhaft zu realisieren, insbesondere nicht mit vorteilhafter Zerfallszeit und vorteilhaftem Mundgefühl. Des Weiteren hat sich herausgestellt, dass in den im Stand der Technik bekannten Verfahren eine schlichte Erhöhung des Wirkstoffgehalts auch im Hinblick auf die physikalischen Parameter nicht zu realisieren ist, da die Verringerung des Hilfsstoffanteils zu unvorteilhaften Tabletteneigenschaften (z.B. im Hinblick auf Härte und Friabilität) führte.

Des Weiteren wurde festgestellt, dass die Zerfallszeit der im Stand der Technik beschriebenen Schmelztabletten nach längerer Lagerung unerwünscht zunimmt.

Zusammenfassend ist festzustellen, dass die im Stand der Technik vorgeschlagenen Formulierungen Nachteile aufweisen. Aufgabe der Erfindung war es daher, diese Nachteile zu überwinden.

Im Speziellen war es eine Aufgabe der vorliegenden Erfindung, eine Schmelztablette enthaltend Sildenafil bereit zu stellen, die einen Wirkstoffgehalt von mehr als 50 Gew.-% aufweisen kann wie definiert in den Ansprüchen.

Des Weiteren war es eine Aufgabe der Erfindung eine Schmelztablette bereit zu stellen, die nach Einnahme beim Patienten als geschmacklich und seitens des Mundgefühls als nicht störend und nicht unangenehm empfunden wird.

Ferner war es eine Aufgabe der Erfindung, eine orale Darreichungsform bereit zu stellen, die ein gutes Mundgefühl und einen guten Geschmack beim Patienten erzielt (d.h. der Patient sollte keinen unangenehmen, wie z.B. bitteren Geschmack, empfinden und z.B. die Masse an Tablettenmaterial soll als solche nicht so groß sein, dass sie als unangenehm empfunden wird).

Der Ausdruck "Mundgefühl" beschreibt dabei die physikalischen und chemischen Interaktionen einer Substanz oder eines Substanzgemisches (im vorliegenden Fall insbesondere der erfindungsgemäßen Schmelztablette) im Mund. Umfasst werden hierbei die Eindrücke vom ersten Gaumenkontakt über das Zerkauen bis zum Schlucken und dem Nachgeschmack.

Das Mundgefühl kann beispielsweise anhand folgender Kriterien beschrieben werden (mit "Probe" ist eine charakteristische Zusammensetzung, Formulierung oder pharmazeutische finale Dosisform gemeint, enthaltend Wirk- und Hilfsstoff beziehungsweise - stoffe):
o Auskleidung des Mundes: Art und Ausmaß der Beschichtung von Zähnen und Schleimhaut des Mundraumes. Von Interesse ist meist die Auskleidung nach Zerkauen oder Zerfall der Probe, also direkt vor dem Schlucken;
o Dichte: Dichte der Schnittfläche im Sinne einer Dichte der Packung, d.h. Schichtung oder zusammenhängendes Komprimat oder Granulat oder dergleichen aus oder bestehend aus z.B. dem Gemisch gemäß einer Formulierung, nachdem eine Probe oder, wie beschrieben ein Produkt beziehungsweise eine finale Dosisform, mit den Backenzähnen vollständig durchtrennt wurde;
o Elastizität: Fähigkeit der Probe (beziehungsweise des Produktes oder der finalen Dosisform) nach der Krafteinwirkung wieder in die ursprüngliche Form zurückzukehren;
o Härte: Maß für die subjektiv empfundene Kraft, um die Probe um eine definierte Strecke zu deformieren, beziehungsweise zu Bruch zu bringen;
o Feuchtigkeit: Menge bzw Auswirkung einer bestimmten Menge Flüssigkeit, die als solche an der Oberfläche der Probe wahrgenommen wird;
o Feuchtigkeits-Absorption: Menge des durch die Probe aufgenommenen Speichels;
o Gleichmäßigkeit: Beschreibt die Einheitlichkeit einer Probe (Geschmack, Textur, Farbe etc.);
o Granularität: Grad der während des Kauvorganges gefühlten Körnigkeit;
o Haftung: Die Kraft, die benötigt wird, um das zu testende Material von einer bestimmten Oberfläche (Lippen, Zähne, Gaumen etc.) zu entfernen;
o Rauheit: Grad des gefühlten Abriebes, den die Probe auf der Zunge hinterlässt;
o Rutschigkeit: Ausmaß, in dem die Probe über die Zunge gleitet;
o Schwere: Beim ersten Kontakt auf der Zunge gefühltes Gewicht der Probe;
o Zerkleinerbarkeit: Die Kraft, die benötigt wird, damit die Probe zerfällt.

Des Weiteren war es eine Aufgabe der Erfindung, eine Schmelztablette bereit zu stellen, die einen schnellen Zerfall im Mund aufweist und auch nach längerer Lagerung den Anforderungen eines schnellen Zerfalls entspricht und die neben den generellen Anforderungen an pharmazeutische Produkte wie Stabilität des Wirkstoffs, keine Verfärbungen oder Zersetzungen oder anderer unerwünschter Veränderungen physikalischer, chemischer oder physiko-chemischer Natur aufweist.

Unter "schneller Zerfall" ist dabei im Rahmen dieser Erfindung eine Zerfallszeit, bestimmt gemäß Ph. Eur. 6.0, Abschnitt 2.9.1 Prüfung A, von weniger als 100 Sekunden, bevorzugt weniger als 50 Sekunden, noch mehr bevorzugt weniger als 30 Sekunden, insbesondere weniger als 25 Sekunden, zu verstehen.

Ferner sollen Schmelztabletten bereitgestellt werden, die sowohl eine vorteilhafte Friabilität als auch eine vorteilhafte Härte aufweisen.

Die Aufgaben konnten durch ein Verfahren zur Herstellung einer Schmelztablette enthaltend Sildenafil-Base, Dochtmittel, Sprengmittel, insbesondere unter Anwendung eines Kompaktierschritts, gelöst werden.

Gegenstand der Erfindung ist daher ein Verfahren zur Herstellung eines pharmazeutischen Intermediats, umfassend die Schritte
(i) Mischen von
   (a-i) Sildenafil-Base,
   (b-i) Dochtmittel,
   (c-i) Sprengmittel,
   (d-i) gegebenenfalls Fließmittel;
(ii) Kompaktieren der Mischung; und
(iii) Zerkleinern des Kompaktats wie in den Ansprüchen definiert.

Ferner ist Gegenstand der Erfindung ein Verfahren zur Herstellung einer Schmelztablette enthaltend Sildenafil-Base, umfassend die Schritte
(I) Herstellen des erfindungsgemäßen Intermediats,
(II) Vermischen des Intermediats mit weiteren pharmazeutischen Hilfsstoffen, und
(III) Verpressen der Mischung aus Schritt (II) zu einer Schmelztablette.

Die Verfahren zur Herstellung des erfindungsgemäßen Intermediats und der erfindungsgemäßen Schmelztablette können somit kombiniert werden. Folglich betrifft die Erfindung auch ein Verfahren zur Herstellung einer Schmelztablette enthaltend Sildenafil-Base, umfassend die Schritte
(I) Herstellen eines Intermediats umfassend die Schritte
   (i) Mischen von
      (a-i) Sildenafil-Base,
      (b-i) Dochtmittel,
      (c-i) Sprengmittel,
      (d-i) gegebenenfalls Fließmittel,
   (ii) Kompaktieren der Mischung, und
   (iii) Zerkleinern des Kompaktats,
(II) Mischen des Intermediats mit weiteren pharmazeutischen Hilfsstoffen, und
(III) Verpressen der Mischung aus Schritt (II) zu einer Schmelztablette.

Des Weiteren ist Gegenstand der Erfindung wie in den Ansprüchen definiert ein durch das erfindungsgemäße Verfahren erhältliches Intermediat und eine durch das erfindungsgemäße Verfahren erhältliche Schmelztablette.

Ferner ist Gegenstand der Erfindung eine Schmelztablette, enthaltend
45 bis 70 Gew.-% Sildenafil-Base,
10 bis 35 Gew.-% Dochtmittel, insbesondere mikrokristalline Cellulose,
5 bis 20 Gew.-% Sprengmittel, insbesondere Crospovidon,
0 bis 5 Gew.-% Fließmittel, insbesondere Siliciumdioxid,
0 bis 10 Gew.-% Schmiermittel, insbesondere Natriumstearylfumarat oder Magnesiumstearat.

Schließlich ist Gegenstand der, wie in den Ansprüchen definiert, die Verwendung von Sildenafil-Base in partikulärer Form, wobei der D50-Wert der Partikelgrößenverteilung 10 bis 55 *µ*m, bevorzugt 20 bis 45 *µ*m, und der D90-Wert der Partikelgrößenverteilung 60 bis 250 *µ*m, bevorzugt 100 bis 200 *µ*m beträgt, zur Herstellung einer Schmelztablette, bevorzugt zur Herstellung einer Schmelztablette mit einer Härte von 30 bis 90 N und mit einer Friabilität von kleiner als 1 %, bevorzugt von 0,01 bis 0,5 %. In einer alternativen bevorzugten Ausführungsform ist Gegenstand der Erfindung die Verwendung von Sildenafil-Base in partikulärer Form, wobei der D50-Wert der Partikelgrößenverteilung 1 bis 25 *µ*m bevorzugt 2 bis 15 *µ*m, und der D90-Wert der Partikelgrößenverteilung 5 bis 80 *µ*m, bevorzugt 10 bis 55 *µ*m beträgt, zur Herstellung einer Schmelztablette, bevorzugt zur Herstellung einer Schmelztablette mit einer Härte von 30 bis 90 N und mit einer Friabilität von kleiner als 1 %, bevorzugt von 0,01 bis 0,5 %.

Die Bestimmung der Partikelgrößenverteilung erfolgt, wie weiter unten ausführlicher erläutert, bevorzugt mit einem an sich bekannten Laserbeugungsmessgerät, angewendet in trockener Meßweise. Die Angaben D10 , D50 und D90 usw. beziehen sich dabei auf die Durchgangssummenkurve, gewichtet mit einem berechneten repräsentativen Durchmesser bzw Volumen der Partikel; so ist D10 die Korngröße, bei der 10% kleiner als der angegebene D-Wert ist, D50 bedeutet 50% sind kleiner , und D90 eben 90% sind kleiner als der angegebene D-Wert.

Wie vorstehend erläutert, betrifft die vorliegende Erfindung ein Verfahren zur Herstellung eines pharmazeutischen Intermediats.

Gemäß der vorliegenden Erfindung versteht man üblicherweise unter einem Intermediat eine pharmazeutische Zusammensetzung, die nicht direkt verabreicht wird, sondern die durch geeignete Verfahren wie beispielsweise Granulieren und/oder Verpressen in eine applizierbare orale Darreichungsform überführt wird.

Das erfindungsgemäße Verfahren zur Herstellung des pharmazeutischen Intermediats umfasst die Schritte (i) Mischen, (ii) Kompaktieren der Mischung und (iii) Zerkleinern des Kompaktats.

Unter dem Begriff "Mischen" versteht man im Rahmen der Erfindung ein Stoffvereinigungsverfahren mit dem Ziel der im Wesentlichen homogenen Verteilung verschiedener Stoffe durch Einwirkung mechanischer Kräfte. Mischen im Sinne der Erfindung erfolgt in üblichen Mischgeräten wie beispielsweise Taumelmischer, Wälzmischer, Schüttelmischer, Freifallmischer, Schermischer, Pflugscharmischer, Planeten-Mischkneter, Z- oder Sigma-Kneter oder Fluid- oder Intensivmischer. Bevorzugt wird ein Taumelmischer verwendet.

Die Mischzeit beträgt im Schritt (i) üblicherweise 1 bis 30 Minuten, bevorzugt 2 Minuten bis 20 Minuten, mehr bevorzugt 5 Minuten bis 17 Minuten.

Wie bereits erwähnt, umfasst Schritt (i) des erfindungsgemäßen Verfahrens das Mischen der Komponenten (a-i) Sildenafil-Base, (b-i) Dochtmittel, (c-i) Sprengmittel, (d-i) gegebenenfalls Fließmittel und gegebenenfalls weitere pharmazeutische Hilfsstoffe.

Insbesondere bei der Herstellung von größeren Chargen hat es sich als vorteilhaft erwiesen, wenn bei der Herstellung des erfindungsgemäßen Intermediats das Dochtmittel (b-i) und das Fließmittel (d-i) vorgemischt werden. Somit ist es bevorzugt, dass in Schritt (i) ein vorgemischtes Gemisch aus Dochtmittel und Fließmittel eingesetzt wird. Ebenfalls können in dem beschriebenen Vormischschritt Süßungsmittel und/oder Aromamittel mit vorgemischt werden. Besonders bevorzugt erfolgt die Vormischung durch ein 5- bis 30-minütiges Vormischen. Durch die Vormischung von Docht- und Fließmittel konnte insbesondere die Gleichförmigkeit des Gehalts positiv beeinflusst werden.

Im Rahmen dieser Erfindung versteht man unter der Komponente (a-i) Sildenafil-Base 1-{[3-(1-Methyl-7-oxo-3-propyl-6,7-dihydro-1H-pyrazolo-[4,3-d]pyrimidin-5-yl)-4-etho-xyphenyl]-sulfonyl}-4-methylpiperazin gemäß vorstehender Formel (1). Die Verbindung gemäß Formel (1) liegt hierbei in Form der freien Base und nicht in Salzform vor.

Die Komponente (a-i) Sildenafil-Base wird in Schritt (i) üblicherweise in einem Anteil von 40 bis 98 Gew.-%, bevorzugt 55 bis 95 Gew.-%, insbesondere 60 bis 90 Gew.-%, bezogen auf das Gesamtgewicht des Intermediats, verwendet.

Unter der Komponente (b-i) Dochtmittel ist im Allgemeinen ein Mittel mit der Fähigkeit, eine biologische Flüssigkeit (bevorzugt Wasser) in einen Feststoff zu ziehen (bevorzugt in die erfindungsgemäße Schmelztablette bzw. in das erfindungsgemäße Intermediat, bevorzugt mittels Physisorption) zu verstehen. Physisorption wird definiert als Form der Adsorption, bei welcher die Flüssigkeitsmoleküle an die Oberfläche des Dochtmittels anhaften können, bevorzugt mittels van-der-Waals-Bindung zwischen der Oberfläche des Dochtmittels und dem adsorbierten flüssigen Molekül (bevorzugt Wasser). Normalerweise bewirkt ein Dochtmittel dies mit oder ohne Aufquellen. Normalerweise wird ein nicht quellendes Dochtmittel, das Wasser anzieht, letztendlich ein Volumen haben, das im Wesentlichen aus dem Volumen des Dochtmittels und dem Wasservolumen besteht, das es anzieht. Im Allgemeinen wird ein quellendes Dochtmittel ein Volumen aufweisen, das im Wesentlichen aus dem Volumen des Dochtmittels, dem Volumen des Wassers, das es anzieht und einem zusätzlichen Volumen, das durch sterische und molekulare Kräfte zwischen den Celluloseketten und sich daran anlagernden Wassermolekülen verursacht wird, besteht. Bevorzugt werden nicht quellende Dochtmittel oder Dochtmittel, deren Quellverhalten in Relation zu dem Wassertransportverhalten untergeordnete Bedeutung auf die im folgenden beschriebene erfindungsgemäße Wirkung aufweisen, verwendet.

Bevorzugt verursacht das Dochtmittel (b-i) in dem erfindungsgemäßen Intermediat oder in der erfindungsgemäßen Tablette die Bildung von Kanälen oder Poren. Dies erleichtert das Eindringen der Wassermoleküle in die Schmelztablette, insbesondere durch Physisorption. Daher besteht die Funktion des Dochtmittels darin, Wasser an die Oberflächen innerhalb der Intermediate zu transportieren, um dadurch Kanäle in oder ein Netzwerk auf einer vergrößerten Oberfläche zu schaffen.

Beispiele von verwendeten Dochtmitteln sind: mikrokristalline Cellulose, silifizierte mikrokristalline Cellulose, Kaolin, Titandioxid, pyrogene Kieselsäure, Aluminium, Niacinamid, m-Pyrol, Bentonit, Magnesium-Aluminium-Silicat, Polyester, Polyethylen, oder Mischungen davon. Bevorzugt enthalten die Dochtmittel der pharmazeutischen Zusammensetzung der vorliegenden Erfindung Cellulose und Cellulosederivate, wie z.B. silifizierte mikrokristalline Cellulose, und Mischungen davon. Besonders bevorzugt wird als Dochtmittel mikrokristalline Cellulose verwendet. Diese ist kommerziell z.B. erhältlich unter dem Handelsnamen Avicel^{®}. Im Rahmen dieser Erfindung wird üblicherweise mikrokristalline Cellulose mit einem gewichtsmittleren Molekulargewicht von 10.000 bis 70.000 g/mol, bevorzugt 25.000 bis 45.000 g/mol, mehr bevorzugt von 30.000 bis 40.000 g/mol verwendet. Dabei wird das gewichtsmittlere Molekulargewicht üblicherweise mittels Gelpermeationschromatographie bestimmt.

Bevorzugt wird dabei üblicherweise eine mikrokristalline Cellulose verwendet, die aus nativer Alpha-Cellulose durch vollständige oder bevorzugt partielle, saure Hydrolyse hergestellt wird. Im Rahmen dieser Erfindung wird üblicherweise mikrokristalline Cellulose mit einem Wassergehalt von < 1,5 Gew.-%, bevorzugt 0,01 bis 1,2 Gew.-%, verwendet. Der Wassergehalt wird im Rahmen dieser Anmeldung bevorzugt mit einem Halogenfeuchtegerät HR83 der Firma Mettler Toledo bei 80 °C und 6 min. Messdauer bestimmt. Üblicherweise wird eine Probe von 3,0 g Gewicht analysiert.

Die Komponente (b-i) Dochtmittel wird in Schritt (i) üblicherweise in einem Anteil von 1 bis 40 Gew.-%, mehr bevorzugt von 5 bis 35 Gew.-%, insbesondere von 10 bis 30 Gew.-%, bezogen auf das Gesamtgewicht der Schmelztablette, verwendet.

Unter der Komponente (c-i) Sprengmittel werden im Allgemeinen ein oder mehrere Stoffe verstanden, die den Zerfall einer Darreichungsform, insbesondere einer Tablette, nach Einbringen in Wasser, beschleunigen. Geeignete Sprengmittel sind z.B. organische Sprengmittel wie Carrageenan, Cellulosen und Cellulosederivate, Croscarmellose, Stärken und Stärkederivate, Natriumcarboxymethylstärke, Polysaccharide, Soja-Polysaccharide, Alginate und Crospovidon. Ebenso können alkalische Sprengmittel verwendet werden. Unter alkalischen Sprengmitteln sind Sprengmittel zu verstehen, die beim Lösen in Wasser einen pH-Wert von mehr als 7,0 erzeugen. Es können auch Gemische der vorstehend genannten Sprengmittel verwendet werden.

Bevorzugt wird als Sprengmittel Crospovidon verwendet. Im Rahmen dieser Erfindung versteht man unter Crospovidon üblicherweise ein quervernetztes Homopolymer des N-Vinyl-2-pyrrolidon, das auch als quervernetztes Polyvinyl-Polypyrrolidon (PVPP) bezeichnet wird. Crospovidon kann im Allgemeinen gemäß US 2,938,017 hergestellt werden und weist üblicherweise ein gewichtsmittleres Molekulargewicht von größer als 1.000.000 g/mol auf.

Die Komponente (c-i) Sprengmittel wird in Schritt (i) üblicherweise in einem Anteil von 1 bis 30 Gew.-%, mehr bevorzugt von 2 bis 15 Gew.-%, insbesondere von 5 bis 12 Gew.-%, bezogen auf das Gesamtgewicht der Schmelztablette, verwendet.

Grundsätzlich könnte auch ein Copolymer, erhältlich durch Copolymerisation von Methacrylsäure und Divinylbenzol, als Sprengmittel (c-i) verwendet werden. Ein derartiges Copolymer ist unter der Bezeichnung Polacrilin insbesondere in Form des Kaliumsalzes (Polacrilin-Kalium, insbesondere gemäß US Pharmacopeia monographiert) bekannt.

Polacrilin-Kalium kann durch folgende Strukturformel veranschaulicht werden. 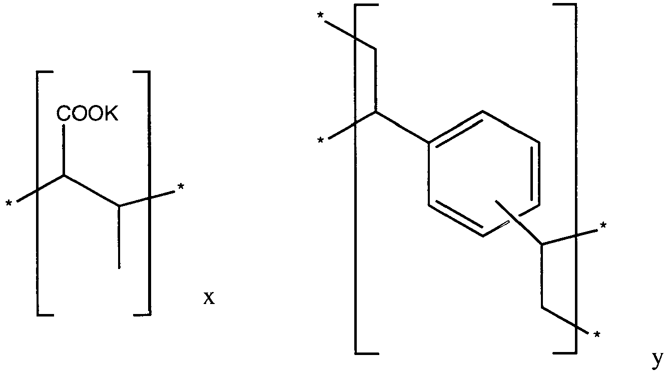 wobei x und y natürliche Zahlen sind, beispielsweise von 10¹ bis 10²⁰, bevorzugt von 10⁶ bis 10¹⁸. Das Verhältnis von x zu y beträgt üblicherweise 50 : 1 bis 1 : 1, bevorzugt 20 : 1 bis 2 : 1, besonders bevorzugt 10 : 1 bis 3 : 1.

Die Verwendung von Polacrilin-Kalium (z.B. in Mengen von 50 Gew.-%) kann jedoch das Mundgefühl negativ beeinträchtigen. Daher enthält die erfindungsgemäße Schmelztablette kein (d.h. 0 Gew.-%) Polacrilin-Kalium.

Unter der Komponente (d-i) Fließmittel sind üblicherweise Mittel zu verstehen, die als Mittel zur Verbesserung der Pulverfließfähigkeit dienen. Fließmittel haben die Aufgabe, in einem Tablettiergemisch sowohl die interpartikuläre Reibung (Kohäsion) zwischen den einzelnen Partikeln als auch das Haften dieser an den Wandflächen der Pressform (Adhäsion) zu vermindern. Ein Beispiel für einen Zusatz zur Verbesserung der Pulverfließfähigkeit ist disperses Siliciumdioxid (z.B. erhältlich als Aerosil^{®}). Bevorzugt wird Siliciumdioxid mit einer spezifischen Oberfläche von 50 bis 400 m²/g, bestimmt nach Gasadsorption gemäß Ph. Eur., 6. Auflage 2.9.26., verwendet.

Die Komponente (d-i) Fließmittel wird in Schritt (i) üblicherweise in einem Anteil von 0 bis 10 Gew.-%, mehr bevorzugt von 0,1 bis 5 Gew.-%, insbesondere von 0,5 bis 3 Gew.-%, bezogen auf das Gesamtgewicht des Intermediats, verwendet.

Weiterhin können in einer bevorzugten Ausführungsform in Schritt (i) die nachstehend genauer erläuterten Süßungsmittel und/oder Aromamittel zugesetzt werden.

Es hat sich unerwartet herausgestellt, dass es zur Lösung der eingangs beschriebenen Aufgaben besonders vorteilhaft ist, wenn im Schritt (i) des erfindungsgemäßen Verfahrens die Einsatzstoffe in einer bestimmten Partikelgrößenverteilung vorliegen. Insbesondere im Hinblick auf das Mundgefühl und den physikalischen Eigenschaften einer resultierenden Schmelztablette kann die Partikelgrößenverteilung wichtig sein, wobei sowohl der D50-Wert (insbesondere für das Mundgefühl) als auch der D90-Wert (insbesondere für physikalische Eigenschaften wie Härte und Friabilität) zu beachten sind.

In einer bevorzugten Ausführungsform wird als Komponente (a-i) deshalb partikuläre Sildenafil-Base eingesetzt, wobei der D50-Wert der Partikelgrößenverteilung der partikulären Sildenafil-Base 1 bis 25 *µ*m, mehr bevorzugt 2 bis 15 *µ*m, beträgt. Der D90-Wert der Partikelgrößenverteilung der partikulären Sildenafil-Base beträgt bevorzugt 5 bis 80 *µ*m, mehr bevorzugt 10 bis 55 *µ*m. Der D10-Wert der Partikelgrößenverteilung der partikulären Sildenafil-Base beträgt bevorzugt 0,1 bis 5 *µ*m, mehr bevorzugt 1,0 bis 4,0 *µ*m.

In einer weiteren bevorzugten Ausführungsform wird (sowohl bei der Herstellung des erfindungsgemäßen Intermediats als auch bei der Herstellung der erfindungsgemäßen Schmelztablette) Dochtmittel (b) und/oder Sprengmittel (c) in partikulärer Form eingesetzt, wobei der D50-Wert der Partikelgrößenverteilung 20 bis 120 *µ*m, mehr bevorzugt 50 bis 110 *µ*m, beträgt. Der D90-Wert der Partikelgrößenverteilung von Dochtmittel und/oder Sprengmittel beträgt bevorzugt 100 bis 300 *µ*m, mehr bevorzugt 125 bis 250 *µ*m.

In einer weiteren bevorzugten Ausführungsform weist in der Partikelgrößenverteilung von Sildenafil-Base, Dochtmittel und/oder Sprengmittel das Verhältnis zwischen dem D90-Wert und D50-Wert (= D90/D50) einen Wert zwischen 1,1 und 25, bevorzugt zwischen 2,0 und 8,0, besonders bevorzugt zwischen 2,5 und 6,0, auf.

In einer weiteren bevorzugten Ausführungsform weist in der Partikelgrößenverteilung von Sildenafil-Base, Dochtmittel und/oder Sprengmittel das Verhältnis zwischen dem D50-Wert und D10-Wert (= D50/D10) einen Wert zwischen 2 und 550, bevorzugt zwischen 5 und 45, besonders bevorzugt zwischen 8 und 12, auf.

In einer weiteren bevorzugten Ausführungsform weist in der Partikelgrößenverteilung von Sildenafil-Base, Dochtmittel und/oder Sprengmittel das Verhältnis zwischen dem D90-Wert und D10-Wert (= D90/D10) einen Wert zwischen 12 und 2500, bevorzugt zwischen 25 und 200, besonders bevorzugt zwischen 40 und 60, auf.

Die "Partikelgröße" wird im Rahmen dieser Erfindung mittels Laserdiffraktometrie bestimmt. Insbesondere wird zur Bestimmung ein Mastersizer 2000 von Malvern Instruments verwendet (Trockenmessung bei 20 °C, dispergiert in Luft, wobei die Auswertung nach dem Fraunhofer Modell erfolgt). Der mittlere Partikeldurchmesser (= die mittlere Partikelgröße), der auch als D50-Wert der integralen Volumenverteilung bezeichnet wird, wird im Rahmen dieser Erfindung als der Partikeldurchmesser definiert, bei dem 50 Volumen-% der Partikel einen kleineren Durchmesser haben als der Durchmesser, der dem D50-Wert entspricht. Ebenso haben dann 50 Volumen-% der Partikel einen größeren Durchmesser als der D50-Wert. Dementsprechend wird der D90-Wert der Partikelgrößenverteilung als die Partikelgröße definiert, bei der 90 Volumen-% der Partikel eine kleinere Partikelgröße aufweisen als die Partikelgröße, die dem D90-Wert entspricht. Analog dazu ist der D10-Wert der Partikelgrößenverteilung des Intermediats als die Partikelgröße definiert, bei der 10 Volumen-% der Partikel eine kleinere Partikelgröße aufweisen als die Partikelgröße, die dem D10-Wert entspricht.

In einer weiteren bevorzugten Ausführungsform des erfindungsgemäßen Verfahrens beträgt das Verhältnis von Gewicht der Komponente (a-i) zu gemeinsamem Gewicht der Komponenten (b-i) und (c-i) 7 : 1 bis 1 : 3, bevorzugt 4 : 1 bis 1 : 2, besonders bevorzugt 3 : 1 bis 1 : 1.

Ferner beträgt in einer weiteren bevorzugten Ausführungsform des erfindungsgemäßen Verfahrens das Gewichtsverhältnis der Komponenten (b-i) zu (c-i) 15 : 1 bis 1 : 2, bevorzugt 10 : 1 bis 1 : 1, besonders bevorzugt 7 : 1 bis 3 : 1.

Bevorzugt weist die aus Schritt (i) erhaltene Mischung einen Wassergehalt von 0,01 bis 4 Gew.-%, bevorzugt 0,1 bis 2 Gew.-%, insbesondere 0,2 bis 1,5 Gew.-% auf. Der Wassergehalt wird dabei mittels oben genannter Halogenfeuchtemethode bestimmt.

Im Schritt (ii) wird die aus Schritt (i) erhaltene Mischung kompaktiert. Im Rahmen dieser Erfindung versteht man unter Kompaktieren üblicherweise das Überführen einer Pulvermischung unter Anwendung von Druck, bevorzugt mechanischem Druck, in Kompaktate, wie z.B. Schülpen oder Bricketts.

Die Kompaktierung kann in üblichen Kompaktiervorrichtungen erfolgen. Bevorzugt wird die Kompaktierung in einem Walzenkompaktierer z.B. Polygran^{®} (Gerteis) durchgeführt. Die Walzkraft beträgt üblicherweise 1 bis 50 kN/cm, bevorzugt 2 bis 30 kN/cm, mehr bevorzugt 3 bis 15 kN/cm. Die Spaltbreite des Walzgranulators beträgt beispielsweise 0,8 bis 6 mm, bevorzugt 1,5 bis 5,0 mm, mehr bevorzugt 2,5 bis 4,0 mm, insbesondere 2,8 bis 3,8 mm.

Die Kompaktierungsbedingungen werden üblicherweise so gewählt, dass das erfindungsgemäße Intermediat in Form eines Kompaktats (Schülpe) vorliegt, wobei die Dichte des Intermediats 0,8 bis 1,3 g/cm³, bevorzugt 0,85 bis 1,20 g/cm³, insbesondere 0,90 bis 1,15 g/cm³, beträgt. Die Werte beziehen sich auf die Scheindichte.

Im Schritt (iii) wird das in Schritt (ii) erhaltene Kompaktat zu einem Granulat zerkleinert. Das Zerkleinern kann dabei üblicherweise mit Hilfe von Zerkleinerungswerkzeugen wie z.B. gegenläufige Stachelwalzen, Sieben oder durch wie oben beschriebene Mischer erfolgen. Bevorzugt werden hierfür Siebe, insbesondere Siebe mit der Maschenweite 0,1 bis 5 mm, bevorzugt 0,5 bis 3 mm, mehr bevorzugt 0,75 bis 2 mm, insbesondere 0,8 bis 1,8 mm, verwendet. Alternativ können auch Mischer, wie z.B. Taumelmischer verwendet werden.

In einer bevorzugten Ausführungsform werden die Zerkleinerungsbedingungen so gewählt, dass die resultierenden Partikel (Granulate) eine Partikelgröße (D50) von 50 bis 400 *µ*m, mehr bevorzugt von 60 bis 250 *µ*m, noch mehr bevorzugt von 90 bis 200 *µ*m, insbesondere von 110 bis 180 *µ*m, aufweisen.

Die resultierenden Intermediate können dann zur Herstellung einer erfindungsgemäßen Schmelztablette verwendet werden. Wie vorstehend erläutert, betrifft also die vorliegende Erfindung des Weiteren ein Verfahren zur Herstellung einer Schmelztablette enthaltend Sildenafil-Base, umfassend die Schritte
(I) Herstellen eines erfindungsgemäßen Intermediats,
(II) Mischen des Intermediats mit weiteren pharmazeutischen Hilfsstoffen, und
(III) Verpressen der Mischung aus Schritt (II) zu einer Schmelztablette.

Unter dem Begriff "Schmelztablette" wird im Rahmen dieser Erfindung eine oral dispersible Tablette verstanden. Somit handelt es sich bei einer Schmelztablette um eine orale, in der Mundhöhle zerfallende Tablette. Während oder nach dem Zerfall können die Bestandteile der erfindungsgemäßen Tablette nicht, teilweise, oder vollständig durch Speichel gelöst werden. Bei der Schmelztablette handelt es sich üblicherweise um eine nicht überzogene Tablette, das heißt nicht beschichtet oder unbefilmt. Insbesondere handelt es sich bei der Schmelztablette nicht um eine Tablette, die mit einem speichelunlöslichen Polymer beschichtete ist, wie sie in US 6,221,402 B1 beschrieben ist.

Gemäß Ph. Eur. 6.0 müssen Schmelztabletten innerhalb von 3 Minuten zerfallen. Im Rahmen dieser Erfindung ist es bevorzugt, dass die erfindungsgemäßen Schmelztabletten eine Zerfallszeit von weniger als 100 Sekunden, mehr bevorzugt von weniger als 50 Sekunden, besonders bevorzugt von weniger als 30 Sekunden, insbesondere von weniger als 25 Sekunden, im Mund aufweisen.

Die Zerfallszeit wird gemäß Ph. Eur. 6.0, Abschnitt 2.9.1 Prüfung A bestimmt. Als Testflüssigkeit wird bevorzugt Wasser verwendet. Sofern im Rahmen dieser Anmeldung auf die durchschnittliche Zerfallszeit Bezug genommen wird, ist darunter der Mittelwert aus der Zerfallszeitbestimmung von 6 Tabletten zu verstehen.

Die erfindungsgemäßen Schmelztabletten enthalten Sildenafil-Base üblicherweise in einer Masse von 15 bis 250 mg, mehr bevorzugt von 20 bis 150 mg. Insbesondere enthalten sie 25 mg, 50 mg oder 100 mg an Sildenafil-Base.

Die erfindungsgemäßen Schmelztabletten weisen üblicherweise ein Gesamtgewicht von weniger als 500 mg auf, mehr bevorzugt von weniger als 350 mg, insbesondere von weniger als 250 mg. Üblicherweise weisen die erfindungsgemäßen Schmelztabletten ein Gewicht von 35 mg, bevorzugt 50 mg oder mehr, insbesondere mehr als 60 mg, auf.

In einer bevorzugten Ausführungsform weisen die erfindungsgemäßen Schmelztabletten einen Anteil von Sildenafil-Base von 45 bis 70 Gew.-%, bevorzugt von 50 bis 65 Gew.-%, mehr bevorzugt von 51 bis 60 Gew.-% auf, bezogen auf das Gesamtgewicht der Schmelztablette.

Bei einem Wirkstoffgehalt von 20 bis 30 mg weisen die erfindungsgemäßen Schmelztabletten üblicherweise ein Gesamtgewicht von weniger als 200 mg auf, mehr bevorzugt von weniger als 150 mg, insbesondere von weniger als 100 mg, auf. Üblicherweise weisen die erfindungsgemäßen Schmelztabletten bei einem Wirkstoffgehalt von 20 bis 30 mg ein Gewicht von mehr als 35 mg, bevorzugt 50 mg oder mehr, insbesondere mehr als 60 mg, auf.

Bei einem Wirkstoffgehalt größer 30 mg und kleiner 70 mg weisen die erfindungsgemäßen Schmelztabletten üblicherweise ein Gesamtgewicht von weniger als 300 mg auf, mehr bevorzugt von weniger als 150 mg, insbesondere von weniger als 120 mg auf. Üblicherweise weisen die erfindungsgemäßen Schmelztabletten ein Gewicht von mehr als 60 mg, bevorzugt 80 mg oder mehr, insbesondere mehr als 85 mg, auf.

Die erfindungsgemäßen Schmelztabletten weisen bei einem Wirkstoffgehalt von 70 mg bis 150 mg üblicherweise ein Gesamtgewicht von weniger als 500 mg auf, mehr bevorzugt von weniger als 350 mg, insbesondere von weniger als 300 mg.

Das Gewichtsverhältnis von Wirkstoff zu Hilfsstoffen beträgt in den erfindungsgemäßen Schmelztabletten üblicherweise 5 : 1 bis 1 : 3, bevorzugt von 3 : 1 bis 1 : 2, insbesondere 2,0 : 1 bis 1 : 1,5.

Die erfindungsgemäßen Schmelztabletten sind daher eindeutig von so genannten "Kautabletten" zu unterscheiden, da diese üblicherweise ein höheres Gewicht (ca. 1,5 bis 3 g) und eine längere Zerfallszeit aufweisen.

Die erfindungsgemäßen Schmelztabletten weisen üblicherweise eine Härte von 25 bis 120 N, bevorzugt von 30 bis 90 N, mehr bevorzugt von 35 bis 60 N, auf. Die Härte wird üblicherweise gemäß Ph. Eur. 6.0, Abschnitt 2.9.8 bestimmt. Sofern im Rahmen dieser Anmeldung auf die mittlere Härte Bezug genommen wird, ist darunter der Mittelwert aus der Härtebestimmung von 10 Tabletten zu verstehen.

Zudem zeigen die resultierenden Tabletten bevorzugt eine Friabilität von kleiner 3 %, besonders bevorzugt von kleiner 1 %, insbesondere kleiner 0,5 %, auf. Die Friabilität wird in einer Apparatur gemäß Ph.Eur. 6.0, Abschnitt 2.9.7 bestimmt, wobei 20 Tabletten für 10 Minuten bei 25 U/min und 25 °C getestet werden.

Die erfindungsgemäßen Schmelztabletten weisen in einer bevorzugten Ausführungsform einen Wassergehalt von < 4,5 Gew.-%, bevorzugt 0,01 bis 3,0 Gew.-%, insbesondere 0,1 bis 1,5 Gew.-% auf, bestimmt gemäß oben erläutertem Halogenfeuchtegerät HR83.

Im Schritt (I) des erfindungsgemäßen Verfahrens zur Herstellung einer Schmelztablette wird, wie oben beschrieben, das erfindungsgemäße Intermediat hergestellt. Das erfindungsgemäße Intermediat bildet dann im erfindungsgemäßen Verfahren zur Herstellung der Schmelztablette die sogenannte "innere Phase". Die Bestandteile der inneren Phase werden auch als "intragranuläre Bestandteile" bezeichnet.

Im Schritt (II) des erfindungsgemäßen Verfahrens zur Herstellung einer Schmelztablette wird das erfindungsgemäße Intermediat mit weiteren pharmazeutischen Hilfsstoffen gemischt. Die in Stufe (II) zugesetzten Hilfsstoffe bilden dann im erfindungsgemäßen Verfahren zur Herstellung der Schmelztablette die sogenannte "äußere Phase". Die Bestandteile der äußeren Phase werden auch als "extragranuläre Bestandteile" bezeichnet.

Weitere pharmazeutische Hilfsstoffe können dabei üblicherweise alle pharmazeutisch üblichen Hilfsstoffe zur Herstellung einer Tablette, insbesondere Schmelztablette, sein.

Der Begriff "Mischen" wird dabei im Rahmen der Erfindung wie oben definiert verstanden.

In einer bevorzugten Ausführungsform handelt es sich bei den in Schritt (II) verwendeten pharmazeutischen Hilfsstoffen um die Komponenten (b-ii) Dochtmittel, (c-ii) Sprengmittel, gegebenenfalls (d-ii) Fließmittel und gegebenenfalls (e-ii) Schmiermittel. Die Bezeichnungen (b-ii), (c-ii), (d-ii) und (e-ii) deuten darauf hin, dass diese Hilfsstoffe zur Bildung der äußeren Phase hinzugefügt werden.

Dabei werden die Komponenten Dochtmittel (b-ii), Sprengmittel (c-ii) und Fließmittel (d-ii) wie oben für die Herstellung des erfindungsgemäßen Intermediats definiert verstanden.

Im Allgemeinen sind Schmiermittel (e-ii) Stoffe, die zur Verringerung der Gleitreibung dienen. Insbesondere soll die Gleitreibung vermindert werden, die beim Tablettieren einerseits zwischen den sich in der Matrizenbohrung auf und ab bewegenden Stempeln und der Matrizenwand sowie andererseits zwischen Tablettensteg und Matrizenwand besteht. Geeignete Schmiermittel stellen z.B. Stearinsäure, Adipinsäure, Natriumstearylfumarat (z.B. PRUV^{®}), Magnesiumstearat und/oder Calciumstearat dar. Bevorzugt wird im Rahmen der Erfindung Natriumstearylfumarat (z.B. PRUV^{®}) als Schmiermittel verwendet. Besonders bevorzugt wird Magnesiumstearat als Schmiermittel verwendet, insbesondere bei Ansatzgrößen von mehr als 5 kg.

Die Komponente (b-ii) Dochtmittel wird in Schritt (II) üblicherweise in einem Anteil von 1 bis 10 Gew.-%, mehr bevorzugt von 2 bis 8 Gew.-%, insbesondere von 3 bis 7 Gew.-%, bezogen auf das Gesamtgewicht der Schmelztablette, verwendet.

Die Komponente (c-ii) Sprengmittel wird in Schritt (II) üblicherweise in einem Anteil von 1 bis 10 Gew.-%, mehr bevorzugt von 2 bis 8 Gew.-%, insbesondere von 3 bis 6 Gew.-%, bezogen auf das Gesamtgewicht der Schmelztablette, verwendet.

Die Komponente (d-ii) Fließmittel wird in Schritt (II) üblicherweise in einem Anteil von 0 bis 10 Gew.-%, mehr bevorzugt von 0,1 bis 5 Gew.-%, insbesondere von 0,5 bis 3 Gew.-%, bezogen auf das Gesamtgewicht der Schmelztablette, verwendet.

Die Komponente (e-ii) Schmiermittel wird in Schritt (II) üblicherweise in einem Anteil von 0 bis 10 Gew.-%, mehr bevorzugt von 0,1 bis 7 Gew.-%, insbesondere von 0,5 bis 5 Gew.-%, bezogen auf das Gesamtgewicht der Schmelztablette, verwendet.

Im erfindungsgemäßen Verfahren beträgt üblicherweise das Gewichtsverhältnis von intragranulärem Dochtmittel (b-i) zu extragranulärem Dochtmittel (b-ii) von 10 : 1 bis 1 : 1, bevorzugt von 5 : 1 bis 2 : 1. Ferner beträgt üblicherweise das Verhältnis von intragranulärem Sprengmittel (c-i) zu extragranulärem Sprengmittel (c-ii) von 5 : 1 bis 1 : 5, bevorzugt von 2 : 1 bis 1 : 2. Ferner beträgt üblicherweise das Verhältnis von intragranulärem Fließmittel (d-i) zu extragranulärem Fließmittel (d-ii) von 5 : 1 bis 1 : 5, bevorzugt von 2 : 1 bis 1 : 2.

In einer bevorzugten Ausführungsform wird in dem erfindungsgemäßen Verfahren zur Herstellung des Intermediats oder in dem erfindungsgemäßen Verfahren zur Herstellung der Schmelztablette als Dochtmittel (b-i) und/oder (b-ii) mikrokristalline Cellulose und/oder als Sprengmittel (c-i) und/oder (c-ii) Crospovidon verwendet.

Als weitere pharmazeutische Hilfsstoffe können gegebenenfalls Trennmittel eingesetzt werden. Unter Trennmittel werden üblicherweise Stoffe verstanden, welche die Agglomeration im Kernbett vermindern. Beispiele sind Talkum, Silicagel, Polyethylenglykol (bevorzugt mit 2000 bis 10.000 g/mol gewichtsmittlerem Molekulargewicht) und/oder Glycerolmonostearat. Bevorzugt wird Talkum verwendet. Trennmittel werden üblicherweise in einer Menge von 0 bis 5 Gew.-%, bevorzugt von 0,1 bis 2 Gew.-%, bezogen auf das Gesamtgewicht der Schmelztablette, verwendet.

Ebenfalls können auch Füllstoffe als weitere pharmazeutische Hilfsstoffe eingesetzt werden. Füllstoffe sind z.B. Calciumphosphate wie Calciumhydrogenorthophosphat, insbesondere in Form des Dihydrats, Calciumcarbonat, Magnesiumcarbonat, Magnesiumoxid, und/oder Calciumsulfat. Die erfindungsgemäße Schmelztablette kann Füllstoffe in der inneren und/oder in der äußeren Phase enthalten.

Des Weiteren können auch Süßungsmittel und/oder Aromamittel als weitere Hilfsstoffe eingesetzt werden. Süßungsmittel und/oder Aromamittel können sowohl in der inneren als auch in der äußeren Phase enthalten sein.

Bevorzugt werden Süßungsmittel verwendet, die eine Süßkraft von 0,2 bis 13.000, bevorzugt von >1 bis 4.000, insbesondere von 10 bis 1.000 haben, bezogen auf die Süßkraft des Rohrzuckers (= 1,0).

Beispiele sind Milchzucker (0,27-0,3), Glycerin (0,5-0,8), D-Glucose (0,5-0,6), Maltose (0,6), Galactose (0,6), Invertzucker (0,8-0,9), Rohrzucker (1,0), Xylit (1,0), D-Fructose (1,0-1,5), Natriumcyclamat (30), D-Tryptophan (35), Chloroform (40), Glycyrrhizin (50), Acesulfam (130). Aspartam (180-200), Dulcin (200), Suosan^{®} (350), Saccharin (Natriumsalz) (400-500), Saccharin (Ammoniumsalz) (600), 1-Brom-5-nitroanilin (700), Naringindehydrochalcon (1000-1500), Thaumatin, Monellin (Peptide) (3000), P-4000, n-Propoxy-2-amino-4-nitrobenzol (4.000), Alitam (3.000) und/oder Neotam (13.000). Der Zahlenwert in Klammern gibt die Süßkraft bezogen auf Rohzucker an. Ebenfalls können Thaumatin und/oder Neohesperidin DC verwendet werden.

In der erfindungsgemäßen Schmelztablette werden Süßungsmittel üblicherweise in einer Menge von 0,1 bis 5 Gew.-%, mehr bevorzugt von 0,5 bis 4 Gew.-%, insbesondere von 0,1 bis 3 Gew.-%, bezogen auf das Gesamtgewicht der Schmelztablette, verwendet.

Die erfindungsgemäßen Schmelztabletten können ferner ein oder mehrere Aromamittel enthalten. Im Rahmen dieser Anmeldung ist der Begriff "Aromamittel" wie in der Richtlinie des Rates 88/388/EWG "Aromastoffe" vom 22. Juni 1988 definiert zu verstehen.

In Schritt (III) des erfindungsgemäßen Verfahrens wird die aus Schritt (II) erhaltene Mischung zu einer Schmelztablette verpresst. Unter "Verpressen" versteht man dabei üblicherweise die Überführung einer Mischung von Stoffen in eine Darreichungsform, bevorzugt Schmelztablette, mit Hilfe einer Tablettiermaschine unter Anwendung von Druck.

Für den Verpressschritt (III) wird im Wesentlichen das erfindungsgemäße Intermediat in granulierter Form mit weiteren, wie nachstehend beschriebenen pharmazeutischen Hilfsstoffen verwendet.

Die Tablettierbedingungen werden dabei bevorzugt so gewählt werden, dass die resultierenden Tabletten ein Verhältnis von Tablettenhöhe zu Gewicht von 0,01 bis 0,1 mm/mg, besonders bevorzugt 0,03 bis 0,06 mm/mg aufweisen.

Als Tablettiermaschinen können die zur Tablettenherstellung üblichen Tablettiermaschinen verwendet werden. Bevorzugt werden Rundläuferpressen oder Exzenterpressen verwendet. Durch die Verwendung einer geeigneten Presskraft können die eingangs erläuterten Aufgaben besonders vorteilhaft gelöst werden, insbesondere im Hinblick auf die physikalischen Eigenschaften (z.B. Härte und Friabilität) der herzustellenden Tabletten. Im Falle von Rundläuferpressen wird üblicherweise eine Presskraft von 0,003 bis 0,40 kN/mm², bevorzugt von 0,006 bis 0,1 kN/mm² verwendet. Die Presskraft wird hierbei als Kraft in Kilonewton pro mm² Tablettenoberfläche bevorzugt senkrecht projizierter Tablettenoberfläche verwendet.

Im Falle von Exzenterpressen wird üblicherweise eine Presskraft von 0,01 bis 0,07 kN/mm² einer Korsch^{®} EKO oder Korsch^{®} XP1 verwendet.

In einer bevorzugten Ausführungsform wird das erfindungsgemäßen Verfahren zur Herstellung des Intermediats und das erfindungsgemäße Verfahren zur Herstellung der Schmelztablette in Abwesenheit von Lösungsmitteln durchgeführt. Unter Lösungsmittel werden dabei üblicherweise Wasser und/oder organische Lösungsmittel, wie z.B. Methanol, Ethanol, Isopropanol verstanden.

Wie bereits erläutert, betrifft die vorliegende Erfindung ein Intermediat, das nach dem vorstehend beschriebenen erfindungsgemäßen Verfahren erhältlich ist. Darüber hinaus betrifft die vorliegende Erfindung eine Schmelztablette, die nach dem vorstehend beschriebenen erfindungsgemäßen Verfahren erhältlich ist.

Somit betrifft die vorliegende Erfindung eine Schmelztablette, bevorzugt erhältlich durch das erfindungsgemäße Verfahren, enthaltend
45 bis 70 Gew.-%, bevorzugt 50 bis 60 Gew.-%, Sildenafil-Base,
10 bis 35 Gew.-%, bevorzugt 20 bis 30 Gew.-%, Dochtmittel, insbesondere mikrokristalline Cellulose,
5 bis 20 Gew.-%, bevorzugt 7 bis 15 Gew.-% Sprengmittel, insbesondere Crospovidon und/oder Stärken bzw. Stärkederivate, insbesondere Natriumcarboxymethylstärke,
0 bis 5 Gew.-%, bevorzugt 1 bis 3 Gew.-%, Fließmittel, insbesondere Siliciumdioxid,
0 bis 10 Gew.-%, bevorzugt 1 bis 5 Gew.-%, Schmiermittel, insbesondere Natriumstearylfumarat.

In einer bevorzugten Ausführungsform der erfindungsgemäßen Schmelztablette beträgt das Verhältnis aus Volumen in cm³ zu Masse an Sildenafil-Base in mg von 1 bis 5 cm³/mg, bevorzugt von 2 bis 4 cm³/mg.

Schließlich ist ein weiterer Gegenstand der vorliegenden Erfindung die Verwendung von Sildenafil, insbesondere Sildenafil-Base, in partikulärer Form, wobei der D50-Wert der Partikelgrößenverteilung 10 bis 55 *µ*m und der D90-Wert der Partikelgrößenverteilung 60 bis 250 *µ*m beträgt, zur Herstellung einer Schmelztablette, bevorzugt zur Herstellung einer Schmelztablette mit einer Härte von 30 bis 90 N, bevorzugt von 40 bis 80 N und einer Friabilität von kleiner 1 %, bevorzugt von 0,01 bis 0,5 %, besonders bevorzugt von 0,05 bis 0,3 %.

Die vorliegende Erfindung ermöglicht somit die Bereitstellung einer Schmelztablette mit vorteilhaft hohem Sildenafil-Gehalt, wobei die Schmelztablette saccharidfrei sein kann. Trotz des hohen Sildenafil-Gehalts zeigt die erfindungsgemäße Tablette ein vorteilhaftes Mundgefühl beim Patienten. Die Erfindung stellt ferner ein technisch vorteilhaftes (z.B. kostengünstiges) Herstellverfahren bereit, das insbesondere auch bei größeren Chargenmengen zu einer vorteilhaften Gleichförmigkeit des Gehalts bei den resultierenden Tabletten führt.

Die Erfindung soll anhand der nachfolgenden Beispiele veranschaulicht werden.

### BEISPIELE

### Herstellung

Zur Herstellung von Schmelztabletten gemäß den Beispielen 1 bis 3 wurde das folgende Herstellungsverfahren verwendet.

Stoffe 1 bis 4 wurden mit Hilfe eines 1 mm Siebes gesiebt und in eine Stahltrommel gefüllt. Mit Hilfe eines Taumelmischers vom Typ Rhoenrad wurden die Stoffe für 10 Minuten gemischt. Danach wurde die Mischung unter Verwendung eines Trockenkompaktors (Typ Gerteis Polygran^{®}) granuliert. Die Stoffe 5 bis 10 wurden mit Hilfe eines 1 mm Siebes gesiebt. Die Stoffe 5 bis 8 wurden dem Granulat beigemischt.

Die so erhaltene Mischung wurde dann für 10 Minuten mit Hilfe eines Taumelmischers vermischt. Talkum und kolloidales Siliciumdioxid wurden hinzugefügt und 5 Minuten vermischt. Das Gleitmittel Natriumstearylfumarat wurde anschließend unter Verwendung eines 0,8 mm Siebes gesiebt. Nachdem das Gleitmittel zur Mischung hinzugegeben wurde, wurde die so erhaltene Mischung für 2 Minuten vermischt. Die zur Tablettierung fertige Mischung wurde unter Verwendung von verschiedenen Kompressionsdrücken (Bsp. 1: 0,025 kN/mm², Bsp. 2a: 0,045 kN/mm², Bsp. 2b: 0,02 kN/mm², Bsp. 3: 0,02 kN/mm²) mit einer Rundläuferpresse zu Tabletten verpresst.

### Beispiel 1

**Tabelle 1: Zusammensetzung zur Herstellung einer Schmelztablette enthaltend Sildenafil-Base 100 mg**

| | **Stärke** | **100 mg** (mg/Dosis) | % |
|---|---|---|---|
| | **Name** | | |
| | **Innere Phase:** | | |
| 1 | Sildenafil-Base | 100,00 | 55,87 |
| 2 | Cellulose, mikrokristalline PH 112 | 40,00 | 22,35 |
| 3 | Crospovidon | 6,00 | 3,35 |
| 4 | Kolloidales Siliciumdioxid | 2,00 | 1,12 |

| | **Äußere Phase:** | | |
|---|---|---|---|
| 5 | Cellulose, mikrokristalline PH 112 | 9,60 | 5,36 |
| 6 | Crospovidon | 9,00 | 5,03 |
| 7 | Natürliches Minzaroma | 2,40 | 1,34 |
| 8 | Aspartam^{®} | 1,00 | 0,56 |
| 9 | Talkum | 2,00 | 1,12 |
| 10 | Kolloidales Siliciumdioxid | 2,00 | 1,12 |
| 11 | Natriumstearylfumarat | 5,00 | 2,79 |
| | **Summe:** | **179,0** | **100,0** |

### Beispiele 2a und 2b:

**Tabelle 2: Zusammensetzung zur Herstellung einer Schmelztablette enthaltend Sildenafil-Base 50/100 mg**

| | **Stärke** | **50 mg** (mg/Dosis) | **100 mg** (mg/Dosis) | % |
|---|---|---|---|---|
| | **Name** | | | |
| | **Innere Phase:** | | | |
| 1 | Sildenafil-Base | 50,00 | 100,00 | 54,95 |
| 2 | Cellulose, mikrokristalline PH 112 | 19,00 | 38,00 | 20,88 |
| 3 | Crospovidon | 4,00 | 8,00 | 4,40 |
| 4 | Kolloidales Siliciumdioxid | 1,00 | 2,00 | 1,10 |

| | **Äußere Phase:** | | | |
|---|---|---|---|---|
| 5 | Cellulose, mikrokristalline PH 112 | 5,40 | 10,80 | 5,93 |
| 6 | Crospovidon | 4,50 | 9,00 | 4,95 |
| 7 | Natürliches Minzaroma | 1,50 | 3,00 | 1,65 |
| 8 | Aspartam^{®} | 0,60 | 1,20 | 0,66 |
| 9 | Talkum | 1,00 | 2,00 | 1,10 |
| 10 | Kolloidales Siliciumdioxid | 1,00 | 2,00 | 1,10 |
| 11 | Natriumstearylfumarat | 3,00 | 6,00 | 3,30 |
| | **Summe:** | **91,0** | **182,0** | **100,0** |

### Beispiel 3

**Tabelle 3: Zusammensetzung zur Herstellung einer Schmelztablette enthaltend Sildenafil-Base 100 mg**

| | **Stärke** | **100** mg (mg/Dosis) |
|---|---|---|
| | **Name** | |
| | **Innere Phase:** | |
| 1 | Sildenafil-Base | 100,00 |
| 2 | Cellulose, mikrokristalline PH 112 | 40,00 |
| 3 | Crospovidon | 6,00 |
| 4 | Kolloidales Siliciumdioxid | 2,00 |

| | **Äußere Phase:** | |
|---|---|---|
| 5 | Cellulose, mikrokristalline PH 112 | 10,80 |
| 6 | Crospovidon | 9,00 |
| 7 | Natürliches Minzaroma | 3,00 |
| 8 | Aspartam^{®} | 1,20 |
| 9 | Talkum | 2,00 |
| 10 | Kolloidales Siliciumdioxid | 2,00 |
| 11 | Natriumstearylfumarat | 8,00 |
| | **Summe** | **184,0** |

### Beispiele 4a und 4b:

Zur Herstellung von Schmelztabletten gemäß Beispiel 4 wurden die Stoffe 1 bis 5 mit Hilfe eines 1 mm Siebes gesiebt und in eine Stahltrommel gefüllt. Mit Hilfe eines Taumelmischers vom Typ Rhoenrad wurden die Stoffe für 10 Minuten gemischt. Danach wurde die Mischung unter Verwendung eines Trockenkompaktors (Typ Gerteis Polygran^{®}) granuliert. Die Stoffe 6 bis 11 wurden mit Hilfe eines 1 mm Siebes gesiebt und dem Granulat beigemischt. Die so erhaltene Mischung wurde dann für 10 Minuten mit Hilfe eines Taumelmischers vermischt. Talkum (12) und kolloidales Siliciumdioxid (13) wurden hinzugefügt und 5 Minuten vermischt. Das Gleitmittel Natriumstearylfumarat (14) wurde anschließend unter Verwendung eines 0,8 mm Siebes gesiebt. Nachdem das Gleitmittel zur Mischung hinzugegeben wurde, wurde die so erhaltene Mischung für 2 Minuten vermischt. Die zur Tablettierung fertige Mischung wurde unter Verwendung von verschiedenen Kompressionsdrücken (Bsp 4a: 0,06 kN/mm², Bsp. 4b: 0,03 kN/mm²) mit einer Rundläuferpresse zu Tabletten verpresst.

**Tabelle 4: Zusammensetzung zur Herstellung einer Schmelztablette enthaltend Sildenafil-Base 50/100 mg**

| | **Stärke** | **50mg** (mg/Dosis) | **100 mg** (mg/Dosis) |
|---|---|---|---|
| | **Name** | | |
| | Trockengranulat: | | |
| 1 | Sildenafil-Base | 50,00 | 100,00 |
| 2 | Mikrokristalline Cellulose | 30,00 | 60,00 |
| 3 | Zweibasiges Calciumphosphat | 16,00 | 32,00 |
| 4 | Natriumstärkeglycolat | 2,00 | 4.00 |
| 5 | Kolloidales Siliciumdioxid | 1,00 | 2,00 |
| | Äußere Phase: | | |
| 6 | Natriumstärkeglycolat | 3,00 | 6,00 |
| 7 | Stärke | 15,65 | 31,30 |
| 8 | Natürliches Minzaroma | 0,30 | 0,60 |
| 9 | Mentholaroma | 0,45 | 0,90 |
| 10 | Zitronenaroma | 1,20 | 2,40 |
| 11 | Saccharin | 0,10 | 0,20 |
| 12 | Talkum | 1,00 | 2,00 |
| 13 | Kolloidales Siliciumoxid | 2,00 | 4,00 |
| 14 | Natriumstearylfumarat | 3,75 | 7,50 |

### Auswertung

In den Beispielen 1 bis 3 konnten Wirkstoffkonzentrationen von größer als 50 Gew.-%, bezogen auf das Gesamtgewicht der Schmelztablette, erreicht werden. Selbst bei einer Menge an Wirkstoff von 100 mg betrug das Gesamtgewicht der Schmelztablette deutlich weniger als 200 mg.

### Friabilität

Die Friabilität für Schmelztabletten gemäß Beispiel 1 betrug 0,25 %. Die Friabilität für Schmelztabletten gemäß Beispiel 2 der Wirkstoffstärke 50 mg (Bsp. 2a) lag bei 0,05 % und für Schmelztabletten der Wirkstoffstärke 100 mg (Bsp. 2b) bei 0,15 %. Schmelztabletten gemäß Beispiel 3 wiesen eine Friabilität von 0,25 % auf.

### Härte

Die mittlere Härte für Schmelztabletten gemäß Beispiel 1 betrug 70 N. Die mittlere Härte betrug für Schmelztabletten gemäß Beispiel 2 der Wirkstoffstärke 50 mg (Beispiel 2a) 50 N und für Schmelztabletten der Wirkstoffstärke 100 mg (Beispiel 2b) 80 N. Ferner lag die mittlere Härte für Schmelztabletten gemäß Beispiel 3 bei 70 N.

### Zerfallszeit

Die durchschnittlichen Zerfallszeiten wurden dabei direkt nach Fertigstellung der Schmelztabletten (Zeitpunkt T₀.), nach 7-tägiger Lagerung der Schmelztabletten (Zeitpunkt T₇) und nach 25-tägiger Lagerung (Zeitpunkt T₂₅) bei 25 °C und 55 % r.F bestimmt.

### Zeitpunkt T₀

Die durchschnittliche Zerfallszeit der gemäß Beispiel 1 hergestellten Schmelztabletten zum Zeitpunkt T₀ betrug 20 sec. Ferner betrug die durchschnittliche Zerfallszeit der gemäß Beispiel 2 hergestellten Schmelztabletten mit einer Wirkstoffstärke von 50 mg zum Zeitpunkt T₀ 10 sec und der gemäß Beispiel 2 hergestellten Schmelztabletten mit einer Wirkstoffstärke von 100 mg zum Zeitpunkt T₀ 35 sec. Des Weiteren betrug die durchschnittliche Zerfallszeit der gemäß Beispiel 3 hergestellten Schmelztabletten zum Zeitpunkt T₀ 25 sec.

### Zeitpunkt T₇ und T₂₅

Es wurde festgestellt werden, dass die durchschnittliche Zerfallszeit der erfindungsgemäßen Schmelztabletten auch nach entsprechender Lagerung nicht signifikant erhöht war.

### Geschmack/Mundgefühl

Da Geschmacksempfindungen und Empfindungen bezüglich des Mundgefühls individuell verschieden sein können, wurden zur Geschmacksprüfung und Prüfung auf Mundgefühl 5 Versuchspersonen herangezogen und das durchschnittliche Geschmacks- und Mundgefühlsempfinden direkt nach Einnahme der gemäß Beispielen 1 bis 3 hergestellten Schmelztabletten ermittelt. Die Geschmacksprüfung und Prüfung auf Mundgefühl erfolgte 1 Stunde nach der letzten Mahlzeit. Die Versuchspersonen waren alle männlich und Nichtraucher. Der Prüfraum war von neutralem Geruch, die Temperatur betrug 20 °C. Die Beurteilung der Reizempfindungen und die Ausbildung der Prüfpersonen erfolgte gemäß DIN 10950. Die Versuchspersonen konnten dabei auf einer Skala von 1 bis 3 Ihre Empfindung bezüglich des Mundgefühls bewerten. Die einzelnen Stufen 1 bis 3 wurden wie folgt definiert: (1) "gutes Mundgefühl", (2) "mittleres Mundgefühl", (3) "schlechtes Mundgefühl. Bezüglich des Mundgefühls wurde für die Schmelztabletten gemäß Beispielen 1 bis 3 von der überwiegenden Anzahl der Versuchspersonen ein "gutes Mundgefühl" festgestellt.

## Patentansprüche

1. Verfahren zur Herstellung einer Schmelztablette enthaltend Sildenafil-Base, umfassend die Schritte
(I) Herstellen eines Intermediats umfassend die Schritte
(i) Mischen von
(a-i) Sildenafil-Base,
(b-i) Dochtmittel,
(c-i) Sprengmittel,
(d-i) gegebenenfalls Fließmittel,
(ii) Kompaktieren der Pulvermischung unter Anwendung von mechanischem Druck, und
(iii) Zerkleinern des Kompaktats,
(II) Mischen des Intermediats mit weiteren pharmazeutischen Hilfsstoffen, und
(III) Verpressen der Mischung aus Schritt (II) zu einer Schmelztablette, wobei die Schmelztablette kein Polacrilin-Kalium enthält.

2. Verfahren gemäß Anspruch 1, wobei als Komponente (a-i) partikuläre Sildenafil-Base eingesetzt wird, wobei der D50-Wert der Partikelgrößenverteilung der partikulären Sildenafil-Base 3 bis 30 *µ*m beträgt und der D90-Wert der Partikelgrößenverteilung der partikulären Sildenafil-Base 5 bis 50 *µ*m beträgt.

3. Verfahren gemäß Anspruch 1 oder 2, wobei das Verhältnis von Gewicht der Komponente (a-i) zu gemeinsamen Gewicht der Komponenten (b-i) und (c-i) 5 : 1 bis 1 : 2 beträgt.

4. Verfahren gemäß einem der Ansprüche 1 bis 3, wobei das Gewichtsverhältnis der Komponenten (b-i) zu (c-i) 10 : 1 bis 1 : 1 beträgt.

5. Verfahren zur Herstellung einer Schmelztablette enthaltend Sildenafil-Base, wobei Sildenafil-Base in einer Menge von 45 bis 70 Gew.-%, bezogen auf das Gesamtgewicht der Schmelztablette, eingesetzt wird.

6. Verfahren gemäß Anspruch 1, wobei in Schritt (II) als pharmazeutische Hilfsstoffe
(b-ii) Dochtmittel,
(c-ii) Sprengmittel,
(d-ii) gegebenenfalls Fließmittel, und
(e-ii) gegebenenfalls Schmiermittel
eingesetzt werden.

7. Verfahren gemäß einem Ansprüche 1 bis 6, wobei das Verfahren in Abwesenheit von Lösungsmitteln durchgeführt wird.

8. Verfahren gemäß einem der Ansprüche 1 bis 7, wobei als Dochtmittel (b-i) und/oder (b-ii) mikrokristalline Cellulose und/oder als Sprengmittel (c-i) und/oder (c-ii) Crospovidon und/oder Natriumcarboxymethylstärke verwendet wird.

9. Verfahren gemäß einem der Ansprüche 1 bis 8, wobei in Schritt (III) die Presskraft 0,01 bis 0,2 kN /mm² beträgt.

10. Schmelztablette erhältlich nach einem Verfahren gemäß einem der Ansprüche 1 bis 9, enthaltend
45 bis 70 Gew.-% Sildenafil-Base,
10 bis 35 Gew.-% Dochtmittel, insbesondere mikrokristalline Cellulose,
5 bis 20 Gew.-% Sprengmittel, insbesondere Crospovidon und/oder Natrium-carboxymethylstärke,
0 bis 5 Gew.-% Fließmittel, insbesondere Siliciumdioxid,
0 bis 10 Gew.-% Schmiermittel, insbesondere Natriumstearylfumarat oder Magnesiumstearat.

11. Schmelztablette gemäß Anspruch 10, wobei das Verhältnis aus Volumen der Schmelztablette in cm³ zu Masse an Sildenafil-Base in mg von 1 bis 5 cm³/mg beträgt.

12. Schmelztablette gemäß einem der Ansprüche 10 oder 11 mit einer Härte von 30 bis 90 N, mit einer Friabilität von 0,01 bis 0,5 %.

13. Verwendung von Sildenafil in partikulärer Form, wobei der D50-Wert der Partikelgrößenverteilung 1 bis 30 *µ*m und der D90-Wert der Partikelgrößenverteilung 5 bis 80 *µ*m beträgt, zur Herstellung einer Schmelztablette erhältlich nach einem Verfahren gemäß einem der Ansprüche 1 bis 9, bevorzugt zur Herstellung einer Schmelztablette mit einer Härte von 30 bis 90 N und mit einer Friabilität von 0,01 bis 0,5 %.

## Claims

1. A process for the preparation of an orodispersible tablet comprising sildenafil base, the process comprising the steps of
(I) preparing an intermediate comprising the steps of
(i) mixing of
(a-i) sildenafil base,
(b-i) a wicking agent,
(c-i) a disintegrant, and
(d-i) optionally a glidant,
(ii) compacting the mixture using mechanical pressure, and
(iii) milling the compacted mixture,
(II) mixing the intermediate further pharmaceutical additives; and
(III) compressing the mixture of step (II) to form an orodispersible tablet, wherein the orodispersible tablet does not contain polacrilin potassium.

2. Process according to claim 1, wherein particular sildenafil base is used as component (a-i), wherein the D50 value of the particle size distribution of the particulate sildenafil base is 3 to 30 *µ*m and the D90 value of the particle size distribution of the particulate sildenafil base is 5 to 50 *µ*m

3. Process according to claim 1 or 2, wherein the ratio of weight of component (a-i) to combined weight of components (b-i) and (c-i) is 5 : 1 to 1 : 2.

4. Process according to any one of claims 1 to 3, wherein the weight ratio of components (b-i) to (c-i) is 10 : 1 to 1 : 1.

5. Process for the preparation of an orodispersible tablet comprising sildenafil base, wherein sildenafil base is used in an amount of 45 to 70 wt % based on the total weight of the orodispersible tablet.

6. Process according to claim 1, wherein
(b-ii) wicking agent,
(c-ii) disintegrant,
(d-ii) optionally a glidant and
(e-ii) optionally a lubricant
are used as pharmaceutical additives in step (II).

7. Process according to any one of claims 1 to 6, wherein the process is carried out in the absence of solvents.

8. Process according to any one of claims 1 to 7, wherein microcrystalline cellulose is used as wicking agent (b-i) and/or (b-ii) and/or crospovidone and/or sodium carboxymethyl starch is used as disintegrant (c-i) and/or (c-ii).

9. Process according to any one of claims 1 to 8, wherein the compaction force in step (III) is 0.01 to about 0.2 kN/mm².

10. An orodispersible tablet obtainable according to the process of any one of claims 1 to 9, comprising
45 to 70 wt % sildenafil base,
10 to 35 wt % wicking agent, in particular microcrystalline cellulose,
5 to 20 wt % disintegrant, in particular crospovidone and/or sodium carboxymethyl starch,
0 to 5 wt % glidant, in particular silicon dioxide,
0 to 10 wt % lubricant, in particular sodium stearyl fumarate or magnesium stearate.

11. Orodispersible tablet according to claim 10, wherein the ratio of the orodispersible tablet in cm³ to mass of sildenafil base in mg is 1 to 5 cm³/mg.

12. Orodispersible tablet according to claim 10 or 11 with a hardness of 30 to 90 N, with a friability of 0.01 to 0.5%.

13. Use of sildenafil in particulate form, wherein the D50 value of the particle size distribution is 1 to 30 *µ*m and the D90 value of the particle size distribution is 5 to 80 *µ*m, for the preparation of an orodispersible tablet obtainable according to the process according to any one of claims 1 to 9, preferably for the preparation of an orodispersible tablet with a hardness of 30 to 90 N and a friability of 0.01 to 0.5%.

## Revendications

1. Procédé pour la manufacture d'un comprimé orodispersible contenant une base de Sildénafil comprenant les étapes:
(I) préparation d'un intermédiaire comprenant les étapes
(i) mélange de
(a-i) base de sildénafil,
(b-i) moyens formant mèche,
(c-i) désintégrant,
(d-i) optionnellement un agent d'écoulement,
(ii) compactage du mélange de poudre sous application de pression mécanique, et
(iii) broyage du matériau compacté,
(II) mélange de l'intermédiaire avec d'autres excipients pharmaceutiques, et
(III) compression du mélange de l'étape (II) en un comprimé orodispersible, dans lequel le comprimé orodispersible ne contient pas de Polacriline de potassium.

2. Le procédé selon la revendication 1, dans lequel une base de sildénafil particulaire est employée en tant que composant (a-i), dans lequel la valeur D50 de la répartition de grandeur de particules de la base de sildénafil particulaire est de 3 à 30 µm et la valeur D90 de la répartition de grandeur de particules de la base de sildénafil particulaire est de 5 à 50 µm.

3. Le procédé selon la revendication 1 ou 2, dans lequel le rapport de la masse du composant (a-i) à la masse des composants (b-i) et (c-i) ensemble est de 5:1 à 1:2.

4. Le procédé selon l'une des revendications 1 à 3, dans lequel le rapport de poids des composants (b-i) à (c-i) est de 10:1 à 1:1.

5. Le procédé pour la manufacture d'un comprimé orodispersible contenant de la base de sildénafil, dans lequel la base de sildénafil est employée en une quantité de 45 à 70 % en poids relatif au poids total du comprimé orodispersible.

6. Le procédé selon la revendication 1, dans lequel
(b-ii) des moyens formant mèche,
(c-ii) des désintégrants,
(d-ii) optionnellement des agents d'écoulement, et
(e-ii) optionnellement des lubrifiants
sont employés en tant qu'excipients pharmaceutiques à l'étape (II).

7. Le procédé selon l'une quelconque des revendications 1 à 6, dans lequel le procédé est exécuté en l'absence de solvants.

8. Le procédé selon l'une des revendications 1 à 7, dans lequel la cellulose micro-cristalline est employée en tant que moyen formant mèche (b-i) et/ou (b-ii) et/ou la crospovidone et/ou le carboxyméthylamidon de sodium est employé en tant que désintégrant (c-i) et/ou (c-ii).

9. Le procédé selon l'une des revendications 1 à 8, dans lequel la force de compression à l'étape (III) est de 0,01 à 0,2 kN/mm².

10. Comprimé orodispersible obtenable selon un procédé selon l'une des revendications 1 à 9, comprenant
45 à 70 % en poids de base de sildénafil,
10 à 35 % en poids de moyens forment mèche, particulièrement de cellulose microcristalline,
5 à 20 % en poids de désintégrant, particulièrement de crospovidone et/ou de carboxyméthylamidon de sodium,
0 à 5 % en poids d'agent d'écoulement, particulièrement de dioxyde de silice,
0 à 10 % en poids de lubrifiant, particulièrement de stéarylfumarate de sodium ou de stéarate de magnésium.

11. Le comprimé orodispersible selon la revendication 10, dans lequel le rapport du volume du comprimé orodispersible en cm³ à la masse de la base de sildénafil en mg est de 1 à 5 cm³/mg.

12. Le comprimé orodispersible selon l'une des revendications 10 ou 11 ayant une dureté de 30 à 90 N, ayant une friabilité de 0,01 à 0,5%.

13. Usage de sildénafil en forme particulaire, dans lequel la valeur D50 de la répartition de grandeur de particules est de 1 à 30 µm est la valeur D90 de la répartition de grandeur de particules est de 5 à 80 µm, pour la manufacture d'un comprimé orodispersible obtenable selon un procédé selon l'une des revendications 1 à 9, préférablement pour la manufacture d'un comprimé orodispersible ayant une dureté de 30 à 90 N et ayant une friabilité de 0,01 à 0,5%.
